# EUROPEAN PATENT SPECIFICATION

(11) **EP 1 204 420 B1**
(45) Date of publication and mention of the grant of the patent: **18.04.2007**
(21) Application number: 00954737.3
(22) Date of filing: 18.08.2000
(51) Int. Cl.: A61K 39/00, A61K 39/002, A61K 39/02, A61P 31/04, A61P 31/10, A61P 33/02

(54) **TREHALOSE PRODUCING PROKARYOTIC CELLS AS VACCINES**
VERWENDUNG VON TREHALOSE PRODUZIERENDEN PROKARYOTISCHEN ZELLEN ALS IMPFSTOFFE
TREHALOSE PRODUISANT DES CELLULES PROKARYOTIQUES, UTILISES EN TANT QUE VACCINS

(30) Priority: 19.08.1999 GB 9919732
(43) Date of publication of application: 15.05.2002
(73) Proprietor: Immunobiology Limited, Cambridge CB2 4AT (GB)
(72) Inventor: COLACO, Camilo, Anthony, Leo, Selwyn, Cambridge CB2 2JN (GB)
(74) Representative: Ouzman, Beverley Nicola Claire
(86) International application number: PCT/GB2000/003223
(87) International publication number: WO 2001/013942

(56) References cited:
- WO-A-98/24882
- US-A- 5 312 909
- BULLIFENT H.L. ET AL: "Stabilisation of Salmonella vaccine vectors by the induction of trehalose biosynthesis." VACCINE, (8 DEC 2000) 19/9-10 (1239-1245). , XP002160646

## Description

This invention relates to the field of vaccines. More specifically, it relates to methods of producing vaccines of trehalose containing procaryotic cells and the compositions obtained thereby.

### BACKGROUND TO THE INVENTION:

Procaryotic cells, particularly bacteria, are widely and increasingly used in medical, agricultural and industrial applications. Agricultural, or environmental, applications include biopesticides and bioremediation. Medical applications include use of bacteria in vaccines as well as for production of pharmaceutical products for other treatments.

For the procaryotic cells to be used effectively, both in terms of desired results and cost, the cells must be able to be stored for significant periods of time whilst preserving their viability. The term viability is used herein to denote that the cells manifest the features of a functioning living organism, such as metabolism and cell division.

Methods for preserving live procaryotic cells suffer from several serious drawbacks, such as being energy-intensive and requiring cold storage. Thus, freeze-drying is often used for preservation and storage of procaryotic cells. However, it has the undesirable characteristic of significantly reducing viability of the cells, as well as being time- and energy-intensive and thus expensive.

PCT WO98/24882 describes a process of stabilising procaryotic cells by the induction of trehalose synthesis and the drying of the resulting cells in a glassy carbohydrate matrix. This process gives stabilised cells that can be stored at ambient temperatures without loss of viability. Trehalose, (α-D-glucopyranosyl-α-D-glucopyranoside), is a naturally occurring, non-reducing disaccharide which was initially found to be associated with the prevention of desiccation damage in certain plants and animals which can dry out without damage and can revive when re-hydrated. Trehalose has been shown to be useful in preventing denaturation of proteins, viruses and foodstuffs during desiccation, see U.S. Patents Nos. 4, 891,319; 5, 149, 653; 5.026, 566; Colaco et al. (1992) *Bio*/*Tech.* 10:1007-1011.

Trehalose synthesis in procaryotic cells is induced by a number of methods including osmotic shock which induces the endogenous production of trehalose, Welsh et al. (1991) *J. Gen. Microbiol.* 137:745-750.

PCT publication No. WO 95/03395 describes a process of improving the viability of bacterial dried cells by the induction of trehalose synthesis by nutrient limitation, heat shock or osmoadaptation. PCT publication No. WO 98/24882 describes a method for the preservation of procaryotic cells by the drying of cells in a carbohydrate matrix after the induction of trehalose synthesis. The latter invention provides compositions of dried cells that can be stored at ambient temperatures and thus enable a number of industrial applications.

Surprisingly, we have now found that the dried, stabilised procaryotic cells produced by the above methods, are more immunogenic than fresh live cells and hence have particular value as the immunogenic determinant active component in vaccine compositions. Furthermore, we have also found that this increased immunogenicity of the stabilised procaryotic cells is not dependent on the drying process considered essential in the above stabilisation processes, but results from the induction of trehalose synthesis. Although more pronounced with dried cells, this increased immunogenicity is also seen in cells induced to produce trehalose but which have not been subjected to a drying process.

### SUMMARY OF THE INVENTION:

The present invention thus provides a method of increasing the immunogenicity of a vaccine composition comprising procaryotic cells as the immunogenic determinant active component, which comprises the steps of:
a. Treating procaryotic cells in vitro under conditions such that an increase of the concentration of trehalose within procaryotic cells is induced by the synthesis of trehalose within the cell;
b. using the induced cells containing trehalose as the immunogenic determinant in the production of a vaccine composition.

Preferably, the treatment of the procaryotic cells is carried out to achieve a concentration of trehalose within the cells of at least 10mM.

Preferably, the induced cells containing the trehalose are dried prior to their use in the production of the vaccine composition, notably in the presence of a non-reducing carbohydrate such as trehalose to provide a storage stable but viable immunogenic determinant for storage prior to use in a vaccine composition.

The invention also provides a method for immunising an animal which comprises administering a pharmaceutically effective amount of a vaccine composition of the invention to an animal sufficient to elicit an immune response in the animal.

Preferably, the vaccine composition contains an adjuvant for the immunogenic determinant, is put up in an aqueous carrier medium and is administered by injection.

The procaryotic cells for use in the present invention are ones which are capable of synthesising trehalose. This ability can be native or can be conferred by recombinant techniques. The ability of a procaryotic cell to synthesise trehalose can be determined by measuring trehalose concentration as described below.

The term procaryotic is used herein to denote cells that exhibit characteristics of procaryotes, which are typically unicellular organisms, lack organelles (such as mitochondria, chloroplasts, and Golgi apparatus), lack a cytoskeleton and lack a discrete nucleus. Examples of procaryotic cells for present use include bacteria, such as eubacteria, cyanobacteria and prochlorophytes; archaebacteria; and other microorganisms such as rickettsias, mycoplasmas, spiroplasmas, and chlamydiae. Preferred procaryotic cells for present use are bacteria.

In general, any procaryotic cell or mixture of cells, particularly bacteria, containing trehalose synthase genes should be capable of synthesising trehalose. Bacteria have two genes involved in trehalose synthesis (i.e. T-Phosphate synthase and T-P phosphatase), whereas yeasts have at least three genes and combinations of these genes may be used to enable trehalose synthesis. Examples of bacteria that contain the trehalose synthase gene include, but are not limited to, Enterobacteriaceae, such as *Salmonella* and *Escherichia (e.g., S. typhimurium* and *E. coli)*; halophilic and halotolerant bacteria, such as *Ectothriorhodospira (e.g., E.halochloris);* micrococcocaceae, such as *Micrococcus (e.g., M.luteus); Rhizobium* species such as *R. japonicum and R. leguminosarum bv phaseoli; Cyanobacteria; Mycobacteria* species such as *M. tuberculosis, M. bovis,* and *M. smegmatis.*

Procaryotic cells can be induced to synthesise trehalose by culturing the cells in vitro under stressful conditions, e.g., osmotic shock, heat or oxygen limitation (shock), carbon/nitrogen starvation, or any combination of the above. Suitable conditions include those heat shock and other conditions described, for example, in PCT publications Nos. WO95/03395 and WO98/24882.

Alternatively, use of inhibitors, such as validomycin, of enzyme(s) such as trahalase involved in trehalose degradation may also result in an increase of trehalose concentration within the cells. Alternatively, the genetic structure of the procaryotic organism may be modified to remove or inhibit that portion of the genetic structure which inhibits or restricts the synthesis of trehalose within the cell so that the cells constitutively synthesise trehalose as they are cultured without the need to apply external stimuli. Such genetic modification can be achieved using any suitable technique. For convenience, the invention will be described hereinafter in terms of the use of external stimuli to induce the production of trehalose within the cell, rather than the use of a procaryotic cell which has had its genetic structure modified.

The term osmotic shock is used herein to denote that the solute concentration in the growth medium within which the cells are cultivated is above the level at which a cell exists and/or grows in its native environment. The solute may be a mixture of salts and the concentration is typically from 0.2 to 0.5 Mols above the level at which the cell is normally cultivated.

We believe that induction of trehalose sythesis under stressful conditions may also induce synthesis or accumulation of other molecules that may be beneficial for preservation, such as betaine and chaperonins or which enhance the vaccine action of the induced cells.

For bacteria, particularly *Escherichia,* trehalose synthesis is preferably induced by growing the cell(s) in conditions of high osmolarity, i.e., salt concentrations sufficient to stimulate trehalose production. To induce trehalose synthesis by osmotic shock, the total concentration of salt(s) in the medium should be at least about 0.2M, preferably at least about 0.4M, more preferably at least about 0.5M. The total concentration of salt(s) should not exceed 0.6M, since above this level trehalose synthesis declines in *E.coli.* The salt concentrations correspond to osmolarities of at least about 350 mOsmoles to about 1.5 Osmoles, preferably at least about 400 mOsmoles to 1 Osmole, most preferably 250 mOsmoles to 500 mOsmoles. Generally, a minimum osmolarity of about 200 mOsmoles is required as this will usually provide a higher concentration of solute than that under which the cells are usually cultivated.

The necessary solute can be provided by the use of a single salt, for example, 200mM NaCl KCl and/or CaCl₂. (NH₄)₂SO₄ may also be used, however only about one half of the amount of trehalose is produced compared to that produced in the presence of KC1, NaCl and/or CaCl₂. A mixture of salts can also be used. In addition, when used to increase the osmolarity of the medium, a non-penetrant solute such as sorbitol and/or glucose can contribute to the stimulation of trehalose synthesis.

The salt concentration (i.e., osmolarity) required to stimulate and/or induce trehalose sythesis will depend upon the genus, species, and/or strain of the procaryotic cell used. Preferably, cell(s) are grown in a minimal medium containing solutes and commercially available minimal media can be supplemented with desired salts and/or other solutes. The use of a minimal medium is not essential and defined media can also be used. The time required to initiate and achieve the desired level of trehalose concentration within the cells will vary depending on the level of osmolarity as well as the genus, species and/or strain of procaryotic cell used. Trehalose synthesis will generally begin within an hour of placing cells in conditions designed to stimulate trehalose production. Generally, in *E. coli* the synthesis of trehalose reaches a maximum at about 15-20 hours.

Synthesis of trehalose may also be stimulated using recombinant methods which are well known in the art. For instance, procaryotic cells can be transfected with a DNA plasmid comprising a DNA sequence encoding the appropriate trehalose synthase gene. The gene in turn is operatively linked to a suitable promoter, which can be constitutive or inducible. Suitable recombinant techniques are described in, for example, Molecular Cloning: A Laboratory Manual, second edition (Sambrook *et al.*, 1989).

The concentration of trehalose synthesised within the procaryotic cells can be measured using any suitable assay technique, for example by high pressure liquid chromatography (HPLC), coupled with electro-chemical detection and glucose assay (Trinder assay using trehalase) for quantitative enzymatic determination of trehalose.

Thin layer chromatography can be used as a qualitative method for the separation of different carbohydrates.

Refractive index detection provides another means of detecting sugars quantitatively.

In measuring trehalose by HPLC, cells are disrupted and trehalose preferentially solubilized in 70% ethanol, followed by removing triglycerides by chloroform extraction. Trehalose concentration is determined by multiplying trehalose concentration (as determined by a standard curve) by the fraction of final volume of supernatant divided by pellet volume. A more detailed description of this assay is provided in Example 1.

Preferably, the synthesis is carried out to provide a concentration of trehalose within the cells of at least about 10mM, for example at least about 30mM, preferably at least about 50mM, notably at least about 100mM.

Thus, in a preferred aspect the invention includes culturing the procaryotic cells under conditions that stimulate intracellular production of trehalose, wherein intracellular concentration of trehalose reaches at least about 10mM, preferably at least about 30mM, more preferably at least about 50mM, notably at least about 100mM. It is particularly preferred that the concentration be at least about 150mM.

The time required for stimulating trehalose synthesis depends, inter alia, on the nature of the procaryotic cells (including genus, species, and/or strain) and the conditions under which trehalose induction occurs (i.e., whether by osmotic shock, oxygen deprivation, etc.). For trehalose induction by osmotic shock, the time required for maximum concentration of trehalose in turn depends on the degree of osmolarity as well as the particular salts used. The optimum conditions for trehalose synthesis can readily be determined by simple trial and errors tests.

The cultivated procaryotic cells containing the intracellular trehalose may then be frozen for storage before use as a vaccine. Alternatively storage of the vaccine can be effected by culturing the procaryotic cells under conditions that increase trehalose concentration to a level effective to increase storage stability, mixing the cells with a drying solution which contains a stabilising agent, and drying the cells under conditions such that a glass is produced having less than about 5% residual moisture. If a killed vaccine rather than a live vaccine is required, the cells may be killed by any suitable method, for example chemical fixation and radiation prior to processing for storage. Though the procaryotic cells may be used as the sole immunogenic determinant active ingredient in the vaccine, an adjuvant may be added in an amount sufficient to enhance the immune response to the procaryotic vaccine. The adjuvant can be added to the procaryotic cells before drying, for example, cholera B toxin sub-unit can be dried simultaneously with *V. cholera.* Alternatively the adjuvant may be obtained and dried separately, and reconstituted along with the procaryotic cells.

Suitable adjuvants include, but are not limited to, aluminium hydroxide, alum, QS-21 (U.S. Pat. No 5,057,540), DHEA (U.S. Pats. Nos. 5,407,684 and 5,077,284) and its derivatives (including salts) and precursors (e.g., DHEA-S), beta-2 microglobulin (WO 91/16924), muramyl dipeptides, muramyl tripeptides (U.S. Pat. No. 5,171,568), monophosphoryl lipid A (U.S. Pat. No. 4, 436, 728; WO 92/16231) and its derivatives (e.g., Detox^{™}), and BCG (U.S. Pat. No.4, 726, 947). Other suitable adjuvants include aluminium salts, squalene mixtures (SAF-1), muramyl peptide, saponin derivatives, mycobacterium wall preparations, mycolic acid derivatives, non-ionic block copolymer surfactants, Quil A, cholera toxin B sub-unit, polyphosphazene and derivatives, and immunostimulating complexes (ISCOMs) such as those described by Takahashi et al. (1990) *Nature* 344:873-875. The choice of an adjuvant will depend in part on the stability of the vaccine in the presence of the adjuvant, the route of administration, and the regulatory acceptability of the adjuvant, particularly when intended for human use. For instance, alum is approved by the United States Food and Drug Administration (FDA) for use as an adjuvant in humans.

The invention also provides a method for treating an animal with a vaccine of the invention by administering a pharmaceutically acceptable quantity of the vaccine of the invention, optionally in combination with an adjuvant, sufficient to elicit an immune response in the animal. The animal is typically a human. However, the invention can also be applied to the treatment of other mammals such as horses, cattle, goats, sheep or swine, and to the treatment of birds, notably poultry such as chicken or turkeys.

The vaccine compositions of the present invention may be administered by any suitable means, such as orally, by inhalation, transdermally or by injection and in any suitable carrier medium. However, it is preferred to administer the vaccine as an aqueous composition by injection using any suitable needle or needle-less technique.

The vaccines of the invention may contain any suitable concentration of the induced procaryotic cells. We prefer that the cells are administered at doses in the range of 10-600 µg, preferably 10-100 µg, most preferably 25 µg, per Kg of body weight of the animal being treated. It will be appreciated that the vaccine of the invention may be applied as an initial treatment followed by one or more subsequent treatments at the same or a different dosage rate at an interval of from 1 to 26 weeks between each treatment to provide prolonged immunisation against the pathogen.

The following examples are provided to illustrate but not limit the invention.

### Example 1: Induction of trehalose in E.coli by osmotic shock:

*E.coli* (NCIMB strain 9484) was cultured in Evans medium (pH 7.0) containing 5mM ammonium chloride. After overnight incubation at 37°C in the initial Evans medium, a 4ml culture of *E.coli* grown in Evans medium under nitrogen limitation was used to inoculate a 200ml culture of Evans medium modified to induce osmotic shock by increasing the salt concentration (KCl) to 0.5M.

Trehalose concentration was measured by high pressure liquid chromatography (HPLC) analysis and significant increases in trehalose concentrations were observed at 15-17 hours after initiation of osmotic shock, with values peaking at less than 20 hours.

### Example 2: Induction of trehalose synthesis in Salmonella:

*Salmonella typhimurium* (1344) was grown overnight at 37°C in M9 (minimal) medium with and without 0.5M NaCl. Cells were harvested by centrifugation and analysed for trehalose concentration by HPLC analysis as described in Example 1. Growth in high salt medium showed at 4 to 5 fold induction of trehalose synthesis as compared to the low salt medium.

### Example 3: Drying of procaryotic cells after induction of trehalose synthesis:

*E.coli* and *Salmonella typhimurium* were grown overnight at 37°C in M9 (minimal) medium with and without 0.5M NaCl and trehalose synthesis induced as described in examples 1 and 2. The induced bacteria were harvested by centrifugation at 10,000 rpm for 10 minutes and the cell pellets resuspended in drying solution containing 45% trehalose, 0.1% cmc (sodium carboxymethyl cellulose, Blanose 7HF, Aqualon) to a typical cell density of 0.5-1.2 x 10⁹ CFU/ml. 300µl and 500µl aliquots were dispensed into 3ml pharmaceutical vials and dried under vacuum without freezing, overnight at ambient temperature and a vacuum pressure of 30mTorr. Alternatively, the aliquots can be freeze-dried using the following protocol: ramp at 2.5°C/min to an initial shelf temperature of -40°C; primary drying was performed at a vacuum pressure of 30mT at -40°C and held for 40 hours; for secondary drying ramp at 0.05°C/min from -40 to 30°C and hold for 12 hours.

### Example 4: Use of induced procaryotic cells as vaccines:

*E.coli* and *Salmonella typhimurium* cells were induced to synthesise trehalose as in Examples 1 and 2 and were used to immunise mice and rabbits. Titration of the bacteria showed that a 100 to 1000 fold lower titre of bacteria induced for trehalose synthesis was required to produce an equivalent antibody response in the animals compared to the use of non-induced bacteria. Dried preparations were generally 2-50 fold more effective on a cell number basis at eliciting protective immunity in the immunised animals than non-dried preparations.

### Example 5: Use of induced procaryotic cells as vaccines; heat-induced trehalose synthesis:

*E.coli* and *Salmonella typhimurium* (strains as in examples 1 and 2) were grown overnight at 37°C in LB medium. 4ml aliquots of the stationary cultures were used to inoculate 200ml of LB medium in a 2 litre conical flask and the cultures grown for 3hrs at 30°C. The log phase cultures were then raised to 40°C and grown for a further 3hrs before the bacteria were harvested by centrifugation at 10,000 rpm for 10 minutes. A similar protocol was used for the growth and induction of *Mycobacterium Bovis* and *Vaccae* (NCTC 11659) which were grown for 2 days in Sauton's medium before dilution to obtain log phase cultures for heat-induction. Cell pellets were resuspended in lysis solution containing 0.5% Tween and the trehalose concentration was measured by high pressure liquid chromatography (HPLC) analysis. Typically 3-5 fold increases in trehalose concentrations were observed as compared to cells grown at 30°C alone.

Bacterial cells induced to synthesise trehalose as described above were killed by repeated freeze-thaw cycles and used to immunise rabbits. Antibody titres in the immunised animals were assayed by 10-fold serial dilutions using a dot-blot assay on total cell lysates prepared as described for trehalose analysis above. Animals vaccinated with induced bacteria showed a 10 to 100 fold higher antibody titre than those immunised with non-induced bacteria.

## Claims

1. A method of increasing the immunogenicity of a vaccine composition comprising procaryotic cells as the immunogenic determinant active component **characterised in that** the method comprises the steps of:
a. treating the procaryotic cells under conditions to induce an increase of the concentration of trehalose within procaryotic cells by synthesis of trehalose within the cell;
b. using the treated procaryotic cells as the immunogenic determinant in the production of a vaccine composition.

2. A method as claimed in claim 1, **characterised in that** the treatment of the procaryotic cells is carried out to achieve a concentration of trehalose within the procaryotic cells of at least 10mM.

3. A method as claimed in claim 1 or claim 2 **characterised in that** the condition causing the synthesis of trehalose within the cells is heat, osmotic shock, suppression of degradation of trehalose, or genetically engineered constitutive synthesis of trehalose within the cells.

4. A method as claimed in any one of the preceding claims, **characterised in that** the induced procaryotic cells containing the trehalose are dried prior to their use in the production of the vaccine composition.

5. A method as claimed in any one of the preceding claims **characterised in that** the procaryotic cells are bacteria.

6. A method as claimed in any one of the preceding claims
**characterised in that** the procaryotic cells are treated by cultivating them in a medium containing one or more solutes and having an osmolarity of at least 350 mOsmoles.

7. A method as claimed in claim 6 **characterised in that** the solute is selected from a sodium, potassium, calcium and / or ammonium salt.

8. A method as claimed in claim 1 **characterised in that** the treatment of the procaryotic cells is carried out to achieve a concentration of trehalose within the procaryotic cells of at least 100mM.

9. A method as claimed In any one of the preceding claims, **characterised in that** the procaryotic cells containing the induced trehalose are killed prior to being used in the vaccine composition.

## Patentansprüche

1. Ein Verfahren zum Erhöhen der Immunogenität einer lmpfstoffzusammensetzung, die prokaryotische Zellen als den immunogenen bestimmenden Wirkstoff beinhaltet, **dadurch gekennzeichnet, dass** das Verfahren die folgenden Schritte beinhaltet:
a. Behandeln der prokaryotischen Zellen unter Bedingungen, so dass eine Erhöhung der Konzentration von Trehalose innerhalb prokaryotischer Zellen durch Synthese von Trehalose innerhalb der Zelle induziert wird;
b. Verwenden der behandelten prokaryotischen Zellen als den immunogenen bestimmenden Faktor bei der Produktion einer Impfstoffzusammensetzung.

2. Verfahren gemäß Anspruch 1, **dadurch gekennzeichnet, dass** die Behandlung der prokaryotischen Zellen ausgeführt wird, um eine Konzentration von Trehalose innerhalb der prokaryotischen Zellen von mindestens 10 mM zu erhalten.

3. Verfahren gemäß Anspruch 1 oder Anspruch 2, **dadurch gekennzeichnet, dass** die Bedingung, die die Synthese von Trehalose innerhalb der Zellen verursacht, Wärme, osmotischer Schock, Unterdrückung von Degradierung von Trehalose oder gentechnisch manipulierte konstitutive Synthese von Trehalose innerhalb der Zellen ist.

4. Verfahren gemäß einem der vorhergehenden Ansprüche, **dadurch gekennzeichnet, dass** die induzierten, prokaryotischen Zellen, die die Trehalose enthalten, vor ihrer Verwendung bei der Produktion der Impfstoffzusammensetzung getrocknet werden.

5. Verfahren gemäß einem der vorhergehenden Ansprüche, **dadurch gekennzeichnet, dass** die prokaryotischen Zellen Bakterien sind.

6. Verfahren gemäß einem der vorhergehenden Ansprüche, **dadurch gekennzeichnet, dass** die prokaryotischen Zellen dadurch behandelt werden, dass sie in einem Medium kultiviert werden, das einen oder mehrere gelöste Stoffe enthält und eine Osmolarität von mindestens 350 mOsmol aufweist.

7. Verfahren gemäß Anspruch 6, **dadurch gekennzeichnet, dass** der gelöste Stoff aus einem Natrium-, Kalium-, Calcium- und/oder Ammoniumsalz ausgewählt ist.

8. Verfahren gemäß Anspruch 1, **dadurch gekennzeichnet, dass** die Behandlung der prokaryotischen Zellen ausgeführt wird, um eine Konzentration von Trehalose innerhalb der prokaryotischen Zellen von mindestens 100 mM zu erhalten.

9. Verfahren gemäß einem der vorhergehenden Ansprüche, **dadurch gekennzeichnet, dass** die prokaryotischen Zellen, die die induzierte Trehalose enthalten, vor ihrer Verwendung bei der Impfstoffzusammensetzung abgetötet werden.

## Revendications

1. Une méthode pour accroître l'immunogénicité d'une composition de vaccin comprenant des cellules procaryotiques en tant que composant immunogène déterminant actif **caractérisée en ce que** la méthode comprend les étapes de :
a. traiter les cellules procaryotiques dans des conditions pour induire une augmentation de la concentration de tréhalose au sein des cellules procaryotiques par synthèse de la tréhalose au sein de la cellule ;
b. utiliser les cellules procaryotiques traitées en tant que déterminant immunogène dans la production d'une composition de vaccin.

2. Une méthode telle que revendiquée dans la revendication 1, **caractérisée en ce que** le traitement des cellules procaryotiques est effectué pour parvenir à une concentration de tréhalose au sein des cellules procaryotiques d'au moins 10mM.

3. Une méthode telle que revendiquée dans la revendication 1 ou la revendication 2 **caractérisée en ce que** la condition provoquant la synthèse de la tréhalose au sein des cellules est la chaleur, le choc osmotique, la suppression de la dégradation de la tréhalose, ou la synthèse constitutive génétiquement manipulée de la tréhalose au sein des cellules.

4. Une méthode telle que revendiquée dans n'importe laquelle des revendications précédentes, **caractérisée en ce que** les cellules procaryotiques induites contenant la tréhalose sont séchées avant d'être utilisées dans la production de la composition de vaccin.

5. Une méthode telle que revendiquée dans n'importe laquelle des revendications précédentes **caractérisée en ce que** les cellules procaryotiques sont des bactéries.

6. Une méthode telle que revendiquée dans n'importe laquelle des revendications précédentes **caractérisée en ce que** les cellules procaryotiques sont traitées en les cultivant dans un milieu contenant un ou plusieurs solutés et ayant une osmolarité d'au moins 350 mOsmoles.

7. Une méthode telle que revendiquée dans la revendication 6 **caractérisée en ce que** le soluté est sélectionné parmi un sel de sodium, de potassium, de calcium et/ou d'ammonium.

8. Une méthode telle que revendiquée dans la revendication 1 **caractérisée en ce que** le traitement des cellules procaryotiques est effectué pour parvenir à une concentration de tréhalose au sein des cellules procaryotiques d'au moins 100 mM.

9. Une méthode telle que revendiquée dans n'importe laquelle des revendications précédentes, **caractérisée en ce que** les cellules procaryotiques contenant la tréhalose induite sont tuées avant d'être utilisées dans la composition de vaccin.
